(19) Europäisches Patentamt
European Patent Office
Office européen des brevets

(11) Numéro de publication : **0 335 801 B1**

(12) # FASCICULE DE BREVET EUROPEEN

(45) Date de publication du fascicule du brevet : **26.05.93 Bulletin 93/21**

(21) Numéro de dépôt : **89400887.9**

(22) Date de dépôt : **30.03.89**

(51) Int. Cl.$^5$ : **C07B 37/04,** C07C 69/743, C07C 255/00, C07D 213/64, C07F 7/08, A01N 53/00, A23K 1/16

(54) **Nouveau procédé de préparation de dérivés trifluorométhylvinyliques à partir de dérivés halogénovinyliques correspondants.**

Demande divisionnaire 91117775.6 déposée le 30/03/89.

(30) Priorité : **31.03.88 FR 8804260**

(43) Date de publication de la demande : **04.10.89 Bulletin 89/40**

(45) Mention de la délivrance du brevet : **26.05.93 Bulletin 93/21**

(84) Etats contractants désignés : **AT BE CH DE ES FR GB GR IT LI LU NL**

(56) Documents cités :
**EP-A- 0 010 859**
**EP-A- 0 019 787**
**EP-A- 0 061 114**
**US-A- 4 582 921**

(56) Documents cités :
**CHEMISTRY LETTERS, no. 12, décembre 1981, pages 1719-1720; Chem. Soc. of Japan, JP, K. MATSUI et al.: "A convenient trifluromethylation of aromatic halides with sodium trifluoroacetate"**
**Advanced Organic Chemistry, pp. 311 a 315**

(73) Titulaire : **ROUSSEL-UCLAF**
**35, Boulevard des Invalides**
**F-75007 Paris (FR)**

(72) Inventeur : **Bonin, Werner**
**Im Schulzehnten 18**
**W-6233 Kelkheim (DE)**
Inventeur : **Demoute, Jean-Pierre**
**65 Avenue Foch**
**F-93360 Neuilly Plaisance (FR)**
Inventeur : **Tessier, Jean**
**30 Rue Jean Moulin**
**F-94300 Vincennes (FR)**

(74) Mandataire : **Tonnellier, Marie-José et al**
**111, route de Noisy B.P. no 9**
**F-93230 Romainville (FR)**

EP 0 335 801 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de dérivés trifluorométhylvinyliques à partir de dérivés halogénovinyliques correspondants.

L'invention a pour objet un procédé de préparation de dérivés trifluorométhylvinyliques sous toutes leurs formes stéréoisomères possibles ainsi que les mélanges de ces stéréoisomères à partir des dérivés halogénovinyliques correspondants correspondants caractérisé en ce que l'on fait réagir sur lesdits dérivés halogénovinyliques, un sel de l'acide trifluoroacétique en présence de sel cuivreux et obtient ainsi le dérivé recherché.

L'invention a plus particulièrement pour objet un procédé où le sel de l'acide trifluoroacétique utilié est le trifluoroacétate de sodium, ainsi qu'un procédé où le sel cuivreux utilisé est l'iodure cuivreux.

Le procédé de l'invention permet de préparer de nombreux produits trifluorométhylvinyliques et notamment certains dérivés de l'acide 2,2-diméthyl cyclopropanecarboxylique.

L'invention a pour objet un procédé de préparation des produits de formule (I) :

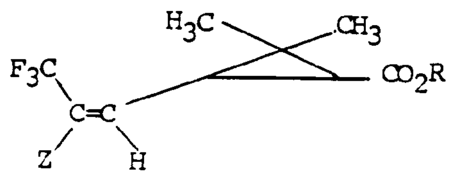

(I)

sous toutes leurs formes stéréoisomères possibles ainsi que les mélanges de ces stéréoisomères, dans laquelle R représente un radical alkyle renfermant jusqu'à 8 atomes de carbone éventuellement substitué par un radical trialkylsilyle ou un radical benzyle éventuellement substitué, par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le reste méthylènedioxy et les atomes d'halogène et Z représente un groupement électroattracteur, caractérisé en ce que l'on soumet un produit de formule (II) :

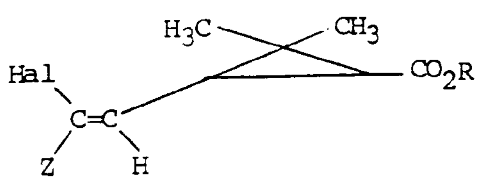

(II)

sous toutes ses formes stéréoisomères possibles ainsi que les mélanges de ces stéréoisomères dans laquelle Z et R conservent leur signification précédente et Hal représente un atome de brome, de chlore ou d'iode à l'action du trifluoroacétate de sodium en présence d'iodure cuivreux pour obtenir le produit de formule (I) correspondant.

Le procédé de l'invention présente l'avantage de conserver la stéréospécificité des produits halogénovinyliques de départ utilisés.

Si la géométrie de la double liaison du produit halogénovinylique utilisé est E, la géométrie du produit trifluorométhyle obtenu est E.

Si la géométrie de la double liaison du produit halogénovinylique est Z, la géométrie du produit trifluorométhylvinylique obtenu est Z.

Dans un mode de réalisation préféré du procédé de l'invention Hal représente un atome de brome.

L'invention a plus particulièrement pour objet un procédé de préparation des produits de formule (I) sous toutes leurs formes stéréoisomères possibles ainsi que les mélanges de ces stéréoisomères, caractérisé en ce que Z représente un radical aryle éventuellement substitué par un atome d'halogène, comme par exemple un radical phényle substitué par un atome de chlore en position 4.

L'invention a également pour objet un procédé de préparation des composés de formule (I) sous toutes leurs formes stéréoisomères possibles ainsi que les mélanges de ces stéréoisomères, caractérisé en ce que Z représente un radical $CO_2R'$, R' représentant un radical alkyle renfermant de 1 à 8 atomes de carbone éventuellement substitué. Comme valeurs préférées de R' on peut citer les radicaux méthyle, éthyle, n-propyle ou isopropyle.

L'invention a tout particulièrement pour objet un procédé de préparation des produits de formule (I) sous toutes leurs formes stéréoisomères possibles ainsi que les mélanges de ces stéréoisomères, caractérisé en ce que R représente un radical alkyle renfermant jusqu'à 4 atomes de carbone, ou encore un procédé caractérisé en ce que R représente un radical alkyle renfermant jusqu'à 4 atomes de carbone substitué par un radical triméthylsilyle.

Les exemples suivants illustrent l'invention sans toutefois la limiter.

**EXEMPLE 1 : [1R-(1alpha,3béta)]-3-[(Z)-2-(4-chlorophényl) 3,3,3-trifluoro 1-propényl] 2,2-diméthyl cyclopropanecarboxylate de méthyle**

On dissout 2,95 g de [1R-(1alpha,3béta)]-3-[(Z)-2-bromo 2-(4-chlorophényl) éthényl] 2,2-diméthyl cyclopropane carboxylate de méthyle (préparation 1) et 4,68 g de trifluoroacétate de sodium dans 30 $cm^3$ de N-méthyl pyrrolidone. On ajoute 3,27 g d'iodure de cuivre. On chauffe pendant 6 heures à 160°C environ. On laisse revenir à la température ambiante, filtre, rince et distille. On obtient un résidu que l'on extrait à l'éther isopropylique. On lave, sèche et amène à sec. On obtient 3,94 g de produit que l'on chromatographie sur silice en éluant par le mélange hexane-éther isopropylique (95-5). On obtient 2,22 g de produit recherché.

Spectre RMN $CDCl_3$ :

| | |
|---|---|
| protons de méthyles géminés | 1,25 - 1,35 ppm |
| protons aromatique | 7,13 7,5 ppm |
| proton éthylénique | 5,63 5,80 ppm |
| protons du carbone en 3 du cyclopropane | 2,33 - 2,75 ppm |
| protons du carbone en 1 du cyclopropane | 1,69 - 1,78 ppm |
| protons de $CO_2-CH_3$ | 3,75 ppm. |

**PREPARATION 1 : [1R-(1alpha,3béta)]-3-[(Z)-2-bromo 2-(4-chlorophényl) éthényl] 2,2-diméthyl cyclopropanecarboxylate de méthyle et isomère 1R-[1alpha,3béta(E)] correspondant**

On dissout 7,8 g de (1R trans) 3-formyl 2,2-diméthyl cyclopropanecarboxylate de méthyle, 17,7 g de [bromo (4-chlorophényl) méthyl] phosphonate de diméthyle dans 70 $cm^3$ de tétrahydrofuranne. On refroidit à -40°C et ajoute en 45 minutes 6,16 g de terbutyle de potassium en solution dans 60 $cm^3$ de tétrahydrofuranne. On agite la solution ainsi obtenue pendant 1 heure à -40°/-45°C. On verse sur une solution aqueuse saturée de phosphate acide de sodium, extrait à l'éther isopropylique, lave, sèche et amène à sec. On obtient ainsi 18,7 g de produit que l'on chromatographie sur silice en éluant à l'aide du mélange hexane-éther isopropylique (95-5). On obtient 2,97 g d'isomère Z (17 %).

Spectre RMN $CDCl_3$ 60 MHz :

| | |
|---|---|
| protons aromatiques | 7,16 à 7,68 ppm |
| proton vinylique | 5,87 - 6 ppm |
| protons des méthyles géminés | 1,23 - 1,35 ppm |
| protons de $CO_2-CH_3$ | 3,7 ppm |
| protons en 1 du cyclopropane | 1,63 - 1,73 ppm |
| protons en 3 du cyclopropane | 2,35 - 2,56 ppm |

On obtient d'autre part 5,91 g d'isomère (34,5 %).

Spectre RMN $CDCl_3$ 60 MHz :

| | |
|---|---|
| protons aromatiques | 7,32 ppm |
| proton vinylique | 5,85 - 5,98 ppm |
| protons des méthyles géminés | 1,17 - 1,22 ppm |
| protons en 1 du cyclopropane | 1,52 ppm |
| protons en 3 du cyclopropane | 1,87 - 2,1 ppm |

**EXEMPLE 2 : [1R-(1alpha,3béta)]-3-[(Z) 2-(4-chlorophényl) 3,3,3-trifluoro 1-propényl] 2,2-diméthyl cyclopropanecarboxylate de 2-(triméthylsilyl) éthyle**

On dissout 3,06 g de [1R-(1alpha,3béta)] 3-[(Z)-2-bromo 2-(chlorophényl) éthényl] 2,2-diméthyl cyclopropanecarboxylate de 2-(triméthyl silyl) éthyle (préparation 2) et 3,86 g de trifluoroacétate de sodium dans 30 $cm^3$ de N-méthyl pyrrolidone. On ajoute 2,7 g d'iodure de cuivre. On maitient le mélange réactionnel pendant 6 heures à 160°C. On laisse revenir à la température ambiante, filtre, rince, élimine les impuretés, extrait à l'éther isopropylique, dilue à l'hexane, lave à l'aide d'une solution saurée de chlorure de sodium, sèche sur sulfate de sodium et amène à sec. On récupère 3,3 g de produit que l'on chromatographie sur silice en éluant par le mélange hexane-toluène (6-4). On isole 1,69 g de produit recherché que l'on rechromatographie en éluant à l'aide du mélange hexane-éther isopropylique (95-5). On obtient ainsi 1,35 g de produit attendu.

Spectre RMN $CDCl_3$ ppm :

| | |
|---|---|
| protons aromatiques | 7,08 à 7,42 ppm |
| proton vinylique | 5,62 - 5,8 ppm |
| protons des méthyles géminés | 1,22 - 1,32 ppm |
| protons du carbone en 1 du cyclopropane | 1,62 - 1,72 ppm |
| protons du carbone en 3 du cyclopropane | 2,35 à 2,82 ppm |
| protons en alpha du $CO_2$ | 4,05 à 4,33 ppm |
| protons en béta du $CO_2$ | 0,86 à 1,13 ppm |

**PREPARATION 2 : [1R-(1alpha,3béta)]-3-[(Z)-2-bromo 2-(4-chlorophényl) éthényl] 2,2-diméthyl cyclopropanecarboxylate de 2-(triméthyl silyl) éthyle et isomère E correspondants**

On dissout 2,84 g d'acide (1R trans) 2,2-diméthyl 3-formyl cyclopropane carboxylique, 7,5 g de [bromo (4-chlorophényl) méthyl] phosphonate de diméthyle dans 20 $cm^3$ de tétrahydrofuranne. On refroidit à -40°/-45°C et ajoute en 25 minutes une solution de 5 g de terbutylate de potassium dans 25 $cm^3$ de tétrahydrofuranne. On agite la solution obtenue pendant 1 heure à -40°/-45°C. On verse dans une solution aqueuse saturée de phosphate acide de sodium, extrait à l'éther isopropylique, lave, sèche et amène à sec. On obtient 7,3 g de résidu que l'on dissout dans 20 $cm^3$ de chlorure de méthyle. On ajoute 2,85 $cm^3$ de triméthylsilyléthanol. On ajoute ensuite à 0°C environ 4,53 g de dicyclohexylcarbodiimide, 70 mg de 4-diméthylaminopyridine et 15 $cm^3$ de chlorure de méthylène. On agite 5 minutes au bain de glace, puis une heure à la température ambiante. On filtre, rince à l'éther isopropylique et amène à sec. On obtient 9 g de produit que l'on chromatographie à l'aide du mélange hexane-éther isopropylique (95-5). On obtient 3,11 g d'isomère Z.

Spectre RMN $CDCl_3$ :

| | |
|---|---|
| protons aromatiques | 7,15 - 7,52 ppm |
| protons des méthyles géminés | 1,24 - 1,37 ppm |
| proton vinylique | 5,87 - 6 ppm |
| protons du carbone en 3 du cyclopropane | 2,33 - 2,55 ppm |
| protons du carbone en 1 du cyclopropane | 1,60 1,7 ppm |
| protons en alpha du $CO_2$ | 4,05 - 4,33 ppm |
| protons en béta du $CO_2$ | 0,86 à 1,15 ppm |

On obtient également 1,88 g d'isorème E

| | |
|---|---|
| protons aromatiques | 7,32 ppm |
| protons des méthyles géminés | 1,17 1,22 ppm |
| proton vinylique | 5,87 - 6 ppm |
| protons du carbone en 3 du cyclopropane | 1,85 à 2,08 ppm |
| protons du carbone en 1 du cyclopropane | 1,47 - 1,57 ppm |
| protons en alpha du $CO_2$ | 3,96 - 4,25 ppm |
| protons en béta du $CO_2$ | 0,8 - 1,08 ppm |

## Revendications

**1.** Procédé de préparation des produits de formule (I) :

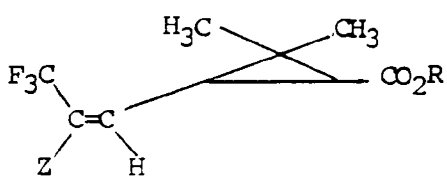

(I)

sous toutes leurs formes stéréoisomères possibles ainsi que les mélanges de ces stéréoisomères, dans laquelle R représente un radical alkyle renfermant jusqu'à 8 atomes de carbone éventuellement substitué par un radical trialkylsilyle ou un radical benzyle éventuellement substitué, par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le reste méthylènedioxy et les atomes d'halogène et Z représente un groupement électroattracteur, caractérisé en ce que l'on soumet un produit de formule (II) :

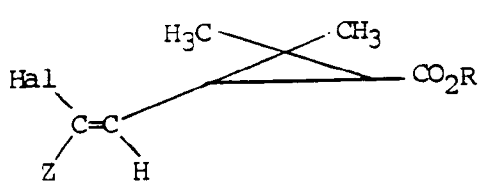

(II)

sous toutes ses formes stéréoisomères possibles ainsi que les mélanges de ces stéréoisomères dans

laquelle Z et R conservent leur signification précédente et Hal représente un atome de brome, de chlore ou d'iode à l'action du trifluoroacétate de sodium en présence d'iodure cuivreux pour obtenir le produit de formule (I) correspondant.

2. Procédé de préparation selon la revendication 1, caractérisé en ce que Hal représnte un atome de brome.

3. Procédé de préparation selon la revendication 1 ou 2, caractérisé en ce que Z représente un radical aryle éventuellement substitué par un atome d'halogène.

4. Procédé de préparation selon la revendication 3, caractérisé en ce que Z représente un radical phényle substitué par un atome de chlore en position 4.

5. Procédé selon la revendication 1 ou 2, caractérisé en ce que Z représente un radical $CO_2R'$, R' représentant un radical alkyle renfermant de 1 à 8 atomes de carbone éventuellement substitué.

6. Procédé selon la revendication 1, caractérisé en ce que R représente un radical alkyle renfermant jusqu'à 4 atomes de carbone.

7. Procédé selon la revendication 1, caractérisé en ce que R représente un radical alkyle renfermant jusqu'à 4 atomes de carbone substitué par un radical triméthylsilyle.

## Claims

1. Preparation process for the products of formula (I):

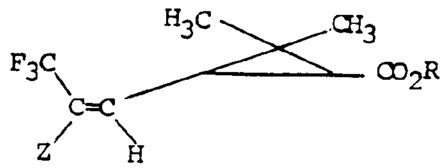

(I)

in all their possible stereoisomer forms as well as the mixtures of these stereoisomers, in which R represents an alkyl radical containing up to 8 carbon atoms optionally substituted by a trialkylsilyl radical or a benzyl radical optionally substituted by one or more radicals chosen from the group constituted by alkyl radicals containing 1 to 4 carbon atoms, alkenyl radicals containing 2 to 6 carbon atoms, alkenyloxy radicals containing 2 to 6 carbon atoms, alkadienyl radicals containing 4 to 8 carbon atoms, the methylenedioxy remainder and halogen atoms and Z represents an electrophilic group, characterized in that a product of formula (II):

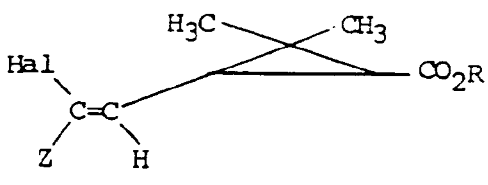

(II)

in all its possible stereoisomer forms as well as the mixtures of these stereoisomers in which Z and R keep their previous meaning and Hal represents a bromine, chlorine or iodine atom, is subjected to the action of sodium trifluoroacetate in the presence of cuprous iodide in order to obtain the corresponding product of formula (I).

2. Preparation process according to claim 1, characterized in that Hal represents a bromine atom.

3. Preparation process according to claim 1 or 2, characterized in that Z represents an aryl radical optionally substituted by a halogen atom.

4. Preparation process according to claim 3, characterized in that Z represents a phenyl radical substituted by a chlorine atom in position 4.

5. Process according to claim 1 or 2, characterized in that Z represents a $CO_2R'$ radical, R' representing an optionally substituted alkyl radical containing 1 to 8 carbon atoms.

6. Process according to claim 1, characterized in that R represents an alkyl radical containing up to 4 carbon atoms.

7. Process according to claim 1, characterized in that R represents an alkyl radical containing up to 4 carbon atoms substituted by a trimethylsilyl radical.

**Patentansprüche**

1. Verfahren zur Herstellung der Produkte der Formel (I)

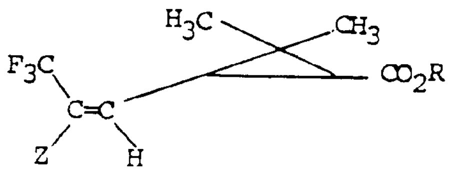

(I)

in sämtlichen ihrer möglichen stereoisomeren Formen sowie der Gemische dieser Stereoisomeren, worin R für einen Alkylrest mit bis zu 8 Kohlenstoffatomen steht, der gegebenenfalls substituiert ist durch einen Trialkylsilylrest oder einen Benzylrest, welcher gegebenenfalls substituiert ist durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen, und Z eine elektronenanziehende Gruppe darstellt, dadurch gekennzeichnet, daß man ein Produkt der Formel (II)

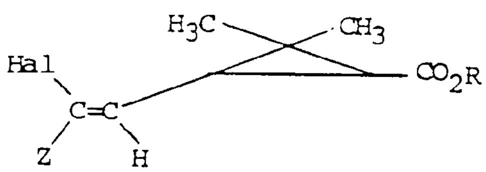

(II)

in sämlichen seiner möglichen stereoisomeren Formen sowie die Gemische dieser Stereoisomeren, worin Z und R die vorstehend angegebene Bedeutung besitzer, und Hal für ein Brom-, Chlor- oder Jodatom steht, der Einwirkung von Natriumtrifluoracetat in Gegenwart von Cuprojodid unterzieht, um zu dem entsprechenden Produkt der Formel (I) zu gelangen.

2. Herstellungsverfahren gemäß Anspruch 1, dadurch gekennzeichnet, daß Hal ein Bromatom bedeutet.

3. Herstellungsverfahren gemäß Anspruch 1 oder 2, dadurch gekennzeichnet, daß Z einen Arylrest bedeutet, der gegebenenfalls durch ein Halogenatom substituiert ist.

4. Verfahren gemäß Anspruch 3, dadurch gekennzeichnet, daß Z einen Phenylrest bedeutet, der in 4-Stellung durch ein Chluoratom substituiert ist.

5. Verfahren gemäß Anspruch 1 oder 2, dadurch gekennzeichnet, daß Z für einen Rest $CO_2R'$ steht, wobei R' einen Alkylrest mit 1 bis 8 Kohlenstoffatomen, der gegebenenfalls substituiert ist, bedeutet.

6. Verfahren gemäß Anspruch 1, dadurch gekennzeichnet, daß R für einen Alkylrest mit bis zu 4 Kohlenstoffatomen steht.

7. Verfahren gemäß Anspruch 1, dadurch gekennzeichnet, daß R für einen Alkylrest mit bis zu 4 Kohlenstoffatomen, der durch einen Trimethylsilylrest substituiert ist, steht.